Europäisches Patentamt

European Patent Office

Office européen des brevets

(19)

(11)     EP 0 664 132 B1

(12)     FASCICULE DE BREVET EUROPEEN

(45) Date de publication et mention
de la délivrance du brevet:
**05.07.2000   Bulletin 2000/27**

(51) Int Cl.⁷: **A61L 24/00**

(21) Numéro de dépôt: **95400093.1**

(22) Date de dépôt: **18.01.1995**

(54) **Composition adhésive, à usage chirurgical, à base de collagène modifié par coupure oxydative et non réticulé**

Klebstoffzusammensetzung für chirurgische Zwecke enthaltend nichtvernetztes und durch oxidativen Abbau modifiziertes Kollagen

Adhesive composition for surgical use based on non-crosslinked collagen modified by oxidative degradation

(84) Etats contractants désignés:
**AT BE CH DE DK ES FR GB GR IE IT LI LU NL PT SE**

(30) Priorité:  **24.01.1994  FR 9400715**

(43) Date de publication de la demande:
**26.07.1995   Bulletin 1995/30**

(73) Titulaire: **Imedex Biomateriaux
69630 Chaponost (FR)**

(72) Inventeurs:
  • **Tardy, Michel
F-69005 Lyon (FR)**
  • **Tiollier, Jérôme
F-69009 Lyon (FR)**
  • **Tayot, Jean-Louis
F-69890 La Tour de Salvagny (FR)**

(74) Mandataire: **Bernasconi, Jean et al
c/o Cabinet Lavoix,
2, Place d'Estienne d'Orves
75441 Paris Cédex (FR)**

(56) Documents cités:
**EP-A- 0 253 715          EP-A- 0 575 273**

## Description

La présente invention se situe dans le domaine des compositions adhésives destinées à un usage chirurgical. D'une manière plus précise, l'invention concerne une composition adhésive biocompatible, biorésorbable, et non toxique, notamment pour la liaison de tissus biologiques, y compris de tissus vivants, entre eux ou pour la liaison de tissus biologiques et d'un biomatériau implanté, à base de collagène ou de gélatine modifié(e) par coupure oxydative et non réticulé(e), soluble en milieu acide.

L'invention concerne également les produits intermédiaires servant à la préparation des compositions adhésives précitées ainsi que leur procédé de préparation et est, en outre, relative à des kits adhésifs destinés à un usage chirurgical extemporané.

Le développement et l'utilisation des colles tissulaires à usage chirurgical a fait l'objet de nombreuses recherches et études expérimentales depuis des décennies.

On connaît, par exemple, une composition adhésive constituée de gélatine, résorcinol et de formaldéhyde (GRF) qui a été utilisée entre les années 1960 et les années 1980. Dans ce composite, la fonction adhésive est attribuée à la gélatine polymérisée par le formaldéhyde, le résorcinol (qui est un phénol) étant utilisé pour réduire la dissolution de la masse adhésive. Des problèmes de toxicité tissulaire aussi bien que de manque d'adhésivité ont été notés dans plusieurs études (Braunwald et al., Surgery, 59 : 1024-1030 (1966) ; Bachet et al., J. Thorac. Cardiovasc. Surg., 83 : 212-217 (1982)). En outre, l'emploi de formaldéhyde dans des compositions à usage chirurgical n'est plus envisageable en raison des risques de relargage de ce composant et de sa toxicité.

On connaît également un système adhésif développé à partir d'un polypeptide isolé du byssus de la moule (marine adhésive protein) (Waite J. H. et al., Biochem., 24 : 5010-5014 (1985)). Originellement extraite à partir de la moule, cette protéine a été ensuite obtenue par voie de synthèse ou de génie génétique (Voir notamment les demandes de brevet européen n° 242 656 et internationale WO 88/03953, et Marumo et al., Biochem. Biophys. Acts, 872, 98-103 (1986) ; Swerdloff M.D. et al., Int. J. Peptide Protein Res., 33, 313 (1989)). Elle est formée d'un enchaînement de motifs répétés constitués d'un décapeptide caractéristique riche en résidus tyrosine et proline hydroxylables et comprenant la DOPA (3,4-dihydroxyphénylalanine) responsables de ces fortes propriétés d'adhésion. Cet adhésif, bien que prometteur au niveau conceptuel, n'est cependant pas disponible suite à des difficultés dans son développement et à la toxicité de certaines formes de décapeptides. Ce système a fait l'objet de nombreux brevets (voir notamment la demande de brevet internationale WO 92/10567 et les demandes de brevet européen publiées sous les n° 243 818 et 244 688 ; les brevets US-A-4 808 702, US-A-4 687 740 et AU-8 824 972).

Mais c'est en fait la mise au point d'une colle à base de fibrinogène et de thrombine qui a permis un réel développement des domaines d'utilisation des colles tissulaires.

L'utilisation du fibrinogène comme biomatériau à visée adhésive remonte au début des années 1940 (Young, Medawar, Lancet II : 126-132 (1940)). Après les premiers résultats obtenus dans les anastomoses des nerfs (Tarlov et Benjamin, Surg. Gynecol. Obstet, 76 366-374 (1943)), ce système adhésif n'a pas apporté satisfaction compte tenu de la faible concentration en fibrinogène des plasmas utilisés.

C'est ensuite dans les années 1970 que le concept d'une colle a été réintroduit par l'utilisation de cryoprécipité contenant des concentrations élevées en fibrinogène (Madras et al., Wien Klin. Wochenschr., 87 : 495-501 (1972)). Ce type de colle a été ensuite commercialisé par la Société IMMUNO sous le nom de "TISSUCOL" (TISSEEL) puis par BEHRINGWERKE sous le nom de "BERIPLAST" et par BIOTRANSFUSION sous le nom de "BIOCOL". Il s'agit d'une solution concentrée de fibrinogène (70-140 mg/ml) contenant du facteur XIII et de la fibronectine dont la polymérisation est induite par une solution de thrombine (4 à 400 Unités Internationales) dans un mélange extemporané. Le fibrinogène polymérise ensuite en fibrine pour reformer un coagulum qui assure l'adhésion des tissus mis en contact.

Les difficultés et problèmes majeurs soulevés par ce produit et ses composants sont d'une part, l'absence de caractérisation complète et de reproductibilité de la quantité de chacun des composants de la solution de fibrinogène (Facteur XIII, fibronectine, aprotinine) ; et d'autre part, la difficulté d'inactivation virale absolue d'un tel produit vis-à-vis des virus non enveloppés comme des Agents Transmissibles Non Conventionnels. Ceci a conduit à une limitation de la possibilité d'utiliser de tels produits à grande échelle, dans certains pays (par exemple les Etats-Unis).

Enfin, il a récemment été décrit dans la demande de brevet européen publiée sous le n° 0 466 383 (Bausch & Lomb Incorporated), une composition adhésive à usage chirurgical constituée de collagène naturel réticulé dont la fluidité et la concentration, nécessaires compte tenu des applications chirurgicales envisagées, sont contrôlées par la proportion de réticulation dans la solution de collagène. Selon ce document, la fluidité et la concentration recherchées sont obtenues en mélangeant une solution de collagène naturel fortement réticulé et une solution de collagène naturel présentant un plus faible degré de réticulation. Des tissus biologiques peuvent être liés par application du mélange précité, préalablement chauffé pour obtenir une fluidité permettant son application sur les tissus, puis en laissant refroidir.

Cependant, ce type de composition adhésive à base de collagène réticulé n'est pas de manipulation aisée et peut poser des problèmes d'application du fait des difficultés à rendre suffisamment fluide cette composition. En effet, plus la proportion de collagène réticulé est importante, plus il est difficile d'atteindre la fluidité requise pour une application correcte sur des tissus biologiques.

Par ailleurs, la présence de collagène faiblement réticulé dans cette composition, bien qu'améliorant la fluidité du mélange et donc la qualité d'application, est une limitation à la résistance mécanique.

On sait, en effet, que la résistance mécanique et la biodégradabilité du collagène dépendent essentiellement du degré de réticulation et de la nature de la réticulation opérée.

On connaît, par ailleurs, par la demande de brevet européen n° 87 401 573.8, un procédé de réticulation du collagène qui permet de le réticuler dans la masse et de manière homogène, sans addition covalente d'une molécule chimique. La réticulation est obtenue en effectuant une oxydation ménagée du collagène à l'aide d'une solution d'acide périodique ou periodate de sodium à température ambiante et en milieu acide, puis en poursuivant le traitement à pH neutre ou basique. Ce procédé permet de préparer des produits collagéniques réticulés, non solubles (gels, films, poudres, lentilles ou encore produits de comblement osseux) présentant de bonnes caractéristiques mécaniques et de biodégradabilité, mais est inadapté à la préparation de produits liquides fluides.

Le but de l'invention est de fournir une composition adhésive biocompatible, biorésorbable et non toxique, adaptée à un usage chirurgical.

Le but de l'invention est encore de fournir une composition adhésive facile à mettre en oeuvre, notamment injectable à l'aide d'aiguilles ou de cathéters, présentant la fluidité requise pour une application correcte notamment sur des tissus biologiques et stable dans le temps tout en conservant ses propriétés adhésives.

Un autre but de l'invention est de fournir une composition adhésive permettant d'obtenir une adhésion satisfaisante parallèlement à une bonne résistance mécanique et biodégradabilité, sans risque de libération de résidus toxiques.

Un autre but de l'invention est de fournir un procédé de préparation d'une telle composition facile à mettre en oeuvre, ainsi que des kits adhésifs à usage chirurgical, d'utilisation simple et pratique.

A cette fin, l'invention à pour objet une composition adhésive à base de collagène non réticulé mais potentiellement réticulable.

La composition adhésive selon l'invention est caractérisée en ce qu'elle comprend une solution acide réactive de collagène ou de gélatine modifié(e) par coupure oxydative, non réticulé(e) et potentiellement réticulable.

L'invention a également pour objet des poudres et solutions acides réactives à base de collagène ou de gélatine, modifié(e) par coupure oxydative, non réticulé(e), qui sont des produits intermédiaires pour la préparation des compositions adhésives précitées, ainsi que leur procédé de préparation.

La poudre est une poudre de collagène ou de gélatine modifié(e) par coupure oxydative, non réticulé(e) et potentiellement réticulable, soluble à pH acide.

Son procédé de préparation est caractérisé en ce qu'il consiste à :

- préparer une solution acide de collagène ;
- soumettre ladite solution aqueuse acide et à température ambiante, à une oxydation ménagée par une solution d'acide périodique ou un de ses sels, à une concentration comprise entre environ 1 et $10^{-5}$ M ;
- précipiter le collagène oxydé et non réticulé, à pH acide et ;
- isoler et concentrer puis déshydrater ledit collagène modifié par coupure oxydative et non réticulé, pour l'obtenir sous forme de poudre acide réactive.

La solution acide réactive est une solution acide concentrée de collagène ou de gélatine modifié(e) par coupure oxydative, non réticulé(e). Elle peut être obtenue soit par concentration directement à partir du précipité redissous résultant de l'oxydation à l'acide périodique, soit à partir de la poudre selon l'invention telle que définie précédemment, après remise en solution dans l'eau, à pH acide.

Son procédé préféré de préparation est caractérisé en ce qu'il consiste à :

- préparer une poudre stérile de collagène ou de gélatine modifié(e) par coupure oxydative, non réticulé(e) et potentiellement réticulable,
- dissoudre dans de l'eau stérile la quantité nécessaire de cette poudre, en chauffant à une température comprise entre environ 40°C et 80°C.

L'invention a encore pour objet des kits adhésifs destinés à un usage chirurgical. Ces kits sont caractérisés en ce qu'ils comprennent :

- une solution acide réactive de collagène ou de gélatine modifié(e) par coupure oxydative, non réticulé(e) et potentiellement réticulable.
- une solution de neutralisation ; et

- des moyens de mélange pour mélanger extemporanément lesdites solutions.

Ils se présentent avantageusement sous la forme d'une double seringue équipée d'un mélangeur.

Enfin, l'invention a pour objet un procédé d'application sur des tissus biologiques et/ou un biomatériau implanté, d'une composition adhésive selon l'invention, caractérisé en ce qu'il consiste à :

- amener la solution acide réactive de collagène ou de gélatine modifié(e) par coupure oxydative et non réticulé(e), à un état de fluidité adapté à une répartition convenable sur lesdits tissus et/ou biomatériau ;
- mélanger, extemporanément, la solution acide réactive avec une solution de neutralisation de manière à obtenir un pH compris entre 6 et 10 ;
- appliquer immédiatement, avant réticulation, le mélange réactif neutralisé et présentant un état de fluidité adapté, sur les tissus biologiques et/ou biomatériau implanté à lier ;
- laisser polymériser par réticulation à la température du corps humain.

Les inventeurs ont découvert, d'une manière surprenante, les propriétés potentiellement adhésives du collagène oxydé.

Ils ont également découvert qu'en effectuant une réticulation "in situ" d'un tel collagène, des tissus biologiques pouvaient être liés entre eux ou avec un biomatériau implanté, cette liaison présentant une résistance mécanique et une biodégradabilité adaptées.

Enfin, les inventeurs ont découvert que le collagène modifié par coupure oxydative et non réticulé, pouvait être conservé à pH acide à l'état congelé, dans le but d'une préparation extemporanée, ce traitement permettant d'éviter l'autoréticulation du collagène tout en préservant ses propriétés réactives adhésives.

Selon l'invention, le terme "collagène" désigne indifféremment le collagène ou la gélatine résultant d'une opération de chauffage ou de toute autre méthode.

Aussi, l'invention fournit un adhésif à base de collagène oxydé, non réticulé, qui a subi un chauffage contrôlé et qui est destiné à être réticulé in situ.

Selon l'invention, on entend par "être réticulé in situ", le fait de provoquer la réticulation du collagène modifié et réactif après application sur des tissus biologiques et/ou diffusion dans lesdits tissus.

Ceci s'oppose à l'enseignement de l'état de la technique qui préconise l'application de collagène naturel déjà réticulé, et qui ne prévoit pas d'opération de réticulation covalente, sans addition d'agent réticulant, après application sur les tissus biologiques.

Le collagène oxydé est obtenu par traitement à l'acide périodique ou l'un de ses sels, qui provoque des coupures dans ledit collagène et crée ainsi des sites réactifs.

Ce traitement est réalisé sur une solution acide de collagène dont la concentration est comprise entre 0,1 et 50 g/l. La concentration en collagène est de préférence de 5 g/l.

Le terme "collagène" désigne ici tout type de collagène qu'il soit d'origine humaine ou d'origine animale tel que par exemple du collagène humain de type I enrichi, de type III enrichi, de type I + III ou de type IV ou encore du collagène animal de type I ou de type I + III.

Le collagène en milieu acide est soumis à l'action de l'acide périodique ou l'un de ses sels par mélange avec une solution dudit acide ou un de ses sels, à une concentration comprise entre 1 et $10^{-5}$ M, de préférence entre 5 $10^{-3}$ M et $10^{-1}$ M.

La réaction est effectuée à température ambiante pendant une durée pouvant aller de 10 minutes à 72 heures et qui est de préférence d'environ 2 heures.

On procède ainsi à une oxydation ménagée du collagène sans en provoquer la réticulation.

L'invention fournit, en outre, un moyen d'isoler et de conserver ce produit intermédiaire sous forme de poudre ou de solution.

En effet, par addition de sel, notamment du chlorure de sodium, à la solution acide précédemment obtenue, on provoque la précipitation du collagène oxydé présentant des sites réactifs utiles pour la réticulation finale.

Après plusieurs lavages successifs, resuspension dans l'éthanol ou l'acétone et déshydratation dans des conditions stériles, on obtient du collagène oxydé sous forme de poudre. Cette poudre est soluble en milieu acide.

La déshydratation peut également être réalisée par lyophilisation, dans des conditions stériles, selon les procédés bien connus et utilisés industriellement dans ce domaine.

Selon un autre mode de réalisation de l'invention, on obtient une solution acide réactive concentrée par concentration du précipité issu de l'oxydation à l'acide périodique et redissolution à chaud ou sous vide pour éliminer les solvants organiques éventuels.

Selon l'invention, la poudre ou la solution concentrée de collagène oxydé potentiellement réactif peut être préparée en relativement grande quantité et être conservée sous cette forme à l'état congelé. La température de conservation est choisie entre -10°C et -80°C environ. Ces conditions de conservation sont simples et permettent, en outre, de préserver la réactivité du collagène modifié.

La poudre et la solution acide concentrée constituent des produits intermédiaires dans la préparation de la composition adhésive selon l'invention.

Conformément à l'invention, on peut préparer une solution acide réactive à partir de la poudre de collagène oxydé non réticulé, potentiellement réticulable, par dissolution en milieu acide.

Pour ce faire, on dissout la poudre de collagène oxydé dans de l'eau stérile chauffée à une température comprise entre 40°C et 80°C. Ce chauffage permet de faciliter la solubilisation du collagène oxydé à des concentrations relativement élevées, qui peuvent s'échelonner de 5 à 30 % en poids.

On procède, de préférence, à une incorporation progressive du collagène oxydé, par exemple par quantité de 5 % (poids/poids) à chaque fois. On attend avantageusement la solubilisation pratiquement complète de chaque quantité de poudre avant d'introduire la quantité suivante.

La rapidité d'incorporation peut varier selon la poudre de collagène oxydé et est généralement comprise entre 10 minutes et 6 heures.

On obtient ainsi une solution homogène.

Selon l'invention, la solution acide réactive de collagène oxydé, non réticulé et potentiellement réticulable, est conservée à l'état congelé, de préférence à une température comprise entre -10°C et -80°C. Cette solution acide réactive est stable dans ces conditions. Elle constitue un produit intermédiaire pour la préparation d'une composition adhésive selon l'invention.

Ce mode de conservation, outre sa simplicité d'application, permet de maintenir la réactivité du collagène jusqu'au moment où son utilisation est souhaitée.

La réticulation du collagène oxydé à pH acide est opérée par simple changement de pH jusqu'à une valeur neutre ou basique. Pour cela, on utilise une solution tampon telle qu'après mélange à la solution acide réactive de collagène oxydé, on obtient un pH compris entre 6 et 10. Elle est avantageusement constituée de carbonate ou de phosphate de sodium.

L'invention permet ainsi de préparer des kits adhésifs destinés à un usage chirurgical, par exemple pour la liaison de tissus entre eux ou avec un biomatériau implanté. Ce kit comprend une solution acide réactive telle que décrit précédemment, une solution de neutralisation, et des moyens de mélange pour mélanger extemporanément ces deux solutions. Le kit est conservé à température négative, de préférence entre -10°C et -80°C afin de préserver la réactivité du collagène oxydé et ses propriétés adhésives.

Selon un mode de réalisation préféré de l'invention, le kit se présente sous la forme d'une double seringue équipée d'un mélangeur, dont l'une contient la solution acide réactive de collagène oxydé tel que décrit précédemment et l'autre le tampon de neutralisation, cette double seringue étant de préférence congelée pour son stockage et sa conservation.

Selon l'invention, la neutralisation est avantageusement effectuée extemporanément avant application de la solution acide réactive de collagène oxydé sur les tissus biologiques à lier et, le cas échéant, le biomatériau implanté.

Le délai nécessaire à la réticulation du mélange réactif ainsi réalisé à partir de la solution acide réactive et de la solution de neutralisation, est suffisant pour permettre l'application de ce mélange réactif sur les tissus et/ou biomatériau, avant polymérisation par réticulation.

Pour une application correcte du mélange neutralisé réactif avec une bonne répartition de celui-ci sur l'ensemble des tissus, ledit mélange est avantageusement rendu suffisamment fluide. Aussi, on réchauffe la solution acide réactive, et la solution de neutralisation provenant par exemple d'un kit tel que précité, à une température comprise entre 30°C et 60°C, de préférence 42° à 50°C.

On réalise ensuite le mélange de ces deux solutions réchauffées à l'aide des moyens de mélange.

On applique immédiatement, avant que la réticulation ne se produise, le mélange réactif neutralisé à cette température, sur les tissus biologiques maintenus dans la position correcte de liaison recherchée.

On provoque alors la réticulation et la liaison des tissus en laissant agir quelques minutes. Le refroidissement éventuel n'est pas nécessaire à la réticulation ni à l'adhésion. Il est imposé par les conditions expérimentales.

On peut, bien sûr, appliquer le mélange neutralisé réactif préalablement amené à la température du corps du patient ou de l'animal.

Le mélange des deux solutions précitées réchauffées et appliquées sur les tissus et/ou un biomatériau, réagit rapidement, par exemple en quelques minutes, pour former un gel adhésif assurant la liaison des tissus entre eux ou avec le biomatériau implanté. Bien entendu, le temps de réticulation est variable en fonction notamment du taux d'oxydation du collagène, de la concentration de la solution acide réactive en collagène oxydé, de la température de réaction, du pH du mélange, etc. Notamment, la réaction est accélérée en augmentant la température ou en utilisant un pH plus alcalin, supérieur à 8.

Selon d'autres modes de mise en oeuvre de l'invention, on peut prévoir d'appliquer, par exemple sur la peau, la solution acide réactive sous forme de spray, puis de laisser la neutralisation s'effectuer par le milieu, ici la peau, en particulier dans le cas où la rapidité d'action de la composition n'est pas essentielle.

On peut également prévoir d'introduire dans le corps du patient ou de l'animal, la solution acide réactive à l'aide de cathéters et, de la même façon, de laisser se réaliser la neutralisation par le milieu organique lui-même. Mais, on peut bien entendu prévoir également d'introduire à l'aide de cathéters le mélange réactif, en particulier si l'effet recherché doit être obtenu très rapidement.

Le matériau adhésif obtenu selon l'invention présente de bonnes caractéristiques mécaniques du fait notamment d'une réticulation homogène et dans la masse à travers les tissus mais également une bonne biodégradabilité.

En outre, il n'est pas toxique car la modification du collagène ne donne pas lieu à l'addition d'un agent chimique étranger susceptible d'être libéré ultérieurement et d'être à l'origine d'une certaine toxicité.

Les compositions et produits selon l'invention présentent plusieurs domaines d'intérêt dans la chirurgie par leurs propriétés adhésives. Chaque domaine et chaque application différente met en oeuvre des fonctions différentes telles que notamment adhésion, hémostase, étanchéité (au liquide ou au gaz), cicatrisation, comblement, prévention des adhérences chirurgicales, embolisation, système local de libération de principes médicamenteux, etc.

La solution acide réactive est un produit fluide pouvant interpénétrer les anfractuosités des tissus biologiques sur lesquels elle est appliquée et permet, après réticulation, de former un gel capable de retenir des tissus apposés côte à côte, en particulier dans le but d'une adhésion et d'une cicatrisation définitive. En outre, l'un des tissus biologiques peut être remplacé par un biomatériau telle qu'une compresse ou un patch.

L'adhésion est une propriété intrinsèque découverte par hasard et la fluidité du produit à appliquer provient avantageusement de la structure moléculaire monomère aussi peu réticulée que possible et stable dans le temps de la solution acide réactive, malgré son caractère potentiellement réactif et réticulable.

Aussi, l'utilisation de la composition selon l'invention peut se substituer, par exemple, à la microsuture des nerfs par le maintien des deux bouts de nerf lésé ou encore servir à l'adhésion des deux parties des tuniques de l'aorte lors des pathologies de dissection aiguë de la crosse aortique.

La grande fluidité de la solution acide réactive ou du mélange réactif selon l'invention, son pouvoir de pénétration des tissus et son aptitude à la réticulation in situ permettent également une utilisation de la composition adhésive selon l'invention dans le but de limiter l'écoulement sanguin, l'écoulement d'un liquide autre que le sang ou l'écoulement d'un gaz, d'une cavité biologique à une autre ou à l'extérieur de l'organisme.

Le matériau adhésif formé après réticulation in situ du collagène oxydé peut constituer une barrière mécanique en plus du rôle que peut jouer également la molécule de collagène vis-à-vis du déclenchement de la cascade de coagulation.

Ces propriétés confèrent un rôle hémostatique à la composition selon l'invention qui peut ainsi servir à l'arrêt du saignement d'une biopsie, d'une incision ou d'une tranche de résection du parenchyme d'un organe hautement vascularisé (foie, rein, rate) par application de la composition seule ou associée soit avec une compresse ou produit hémostatique, soit avec les techniques de ligature ou d'électrocoagulation conventionnelle. Elle peut servir également à l'arrêt du saignement ou suintement diffus de surface de tissus divers après traumatisme, incision ou résection partielle.

La composition selon l'invention peut également assurer, par exemple, l'étanchéité lors des curages du système lymphatique (lymphostase), l'étanchéité des anastomoses réalisées par suture de deux parties d'organes ou de viscères entre eux, en particulier lorsque l'un de ces organes contient des fluides biologiques (cerveau-dure mère, oeil-cornée, appareil digestif, vaisseau) ; l'étanchéité visant à une meilleure cicatrisation de l'anastomose pour assurer une étanchéité définitive des tissus permettant d'éviter la fistulisation (anastomose viscérale, vasculaire, oesophagienne) ; ou encore l'étanchéité des anastomoses pulmonaires difficiles à rendre étanche à la pression des gaz.

La composition selon l'invention apporte une matrice collagénique en elle-même dénuée de principes toxiques, au contraire de la colle GRF précédemment décrite par exemple, qui est capable d'être infiltrée par les cellules et les vaisseaux et permet ainsi d'être remplacée progressivement par le tissu conjonctif endogène. Initialement, le gel réticulé de collagène oxydé procure une matrice qui adhère fermement avec les tissus sous-jacents et assure une barrière mécanique empêchant ainsi toute fuite. Secondairement, le tissu conjonctif formé en place et dans le produit selon l'invention assure l'étanchéité et la réparation définitive.

Le temps de dégradation, et donc l'adhésivité primaire, et le rôle dans le comblement peuvent être modulés par la concentration du collagène dans la solution acide réactive ou par son oxydation en particulier au niveau de la densité des liaisons réticulantes.

La solution acide réactive apporte intrinsèquement, par ses propriétés adhésives, la possibilité de lier un tissu avec un autre ou un biomatériau, par exemple une compresse ou un patch en collagène, et ainsi permet de compléter la matrice nécessaire au comblement et à la cicatrisation de tissus lésés.

La composition selon l'invention peut ainsi être appliquée sur des parois à la suite de résection de tumeur ou de kyste, ou servir notamment au remplissage de cavités évidées lors de curetages ou de résections de tumeur ou de kyste.

Elle peut également être employée dans le but d'une obstruction définitive de la lumière de vaisseaux d'un organe ou d'un tissu afin d'en supprimer ou limiter la vascularisation pour provoquer sa régression ou sa disparition.

La composition selon l'invention peut ainsi s'appliquer notamment à l'embolisation de malformations artério-veineuses cérébrales ou de tumeurs de type méningiome ou hépatique.

Dans le domaine de la prévention des adhérences chirurgicales, la composition selon l'invention permet au tissu lésé de reformer des plans de cicatrisation corrects conduisant à limiter l'apparition ultérieure d'adhérences chirurgicales, c'est-à-dire de liens tissulaires pathologiques inorganisés reliant deux tissus ou deux organes entre eux alors qu'ils sont naturellement séparés, grâce à l'hémostase plus soigneuse, la meilleure étanchéité et la meilleure séparation des plans tissulaires qu'elle procure.

La composition selon l'invention peut ainsi trouver des applications notamment en chirurgie réparatrice des tendons ou dans l'étanchéité des anastomoses digestives pour limiter l'apparition d'adhérences'liées à la tentative de réparation naturelle.

Par ailleurs, la composition selon l'invention peut, parallèllement à ses propriétés adhésives, constituer un système local de libération de principes médicamenteux.

En effet, elle constitue une matrice collagénique pouvant servir de réservoir pour une substance active. La libération de cette substance se déroule alors en deux temps : dans un premier temps, la portion de la substance active en phase soluble dans le fluide interstitiel du gel adhésif est libérée par diffusion de fluides. Dans un deuxième temps, la substance active enchassée ou liée sur les molécules de collagène est relarguée au cours de la dégradation progressive de la matrice.

En conséquence, l'invention peut s'appliquer aux domaines de la chirurgie digestive et générale, de la chirurgie hépatique - gastrique et pancréatique, de la neurochirurgie, de l'urologie et néphrologie, de la chirurgie gynécologique et obstétricale, de la chirurgie oto-rhino-laryngologie, de la chirurgie crânio-faciale, plastique et reconstructrice, de la chirurgie cardio-vasculaire, de la chirurgie pulmonaire et thoracique, en ophtalmologie, orthopédie, chirurgie dentaire-parodontologie, etc.

On remarque à la lecture de ce qui précède que chaque fonction de la composition selon l'invention se retrouve dans plusieurs domaines et applications et lors de chaque application chirurgicale, plusieurs fonctions peuvent être mises en oeuvre ; ces fonctions sont néanmoins hiérarchisées selon qu'elles sont essentielles ou complémentaires ; les premières résultent des propriétés intrinsèques de la composition, à savoir adhésivité, biocompatibilité, biorésorbabilité et non toxicité et sont majeures dans le rôle de la composition selon l'invention, quelle que soit la fonction recherchée ; les secondes découlent desdites propriétés de base.

Les exemples qui suivent sont une illustration du procédé de préparation d'une composition selon l'invention ainsi que de l'isolation des produits intermédiaires à cette préparation. Ils illustrent également un mode de réalisation préféré d'un kit adhésif selon l'invention ainsi que les propriétés adhésives de la composition selon l'invention.

EXEMPLES

## I - PREPARATION.

**Exemple 1 :** Préparation d'une poudre de collagène modifié par coupure oxydative à l'aide d'acide périodique et non réticulé.

Pour préparer 20 g de poudre, on prépare une solution acide de collagène en dissolvant 20 g de collagène I bovin pepsiné (sec et sans cendre) dans 20 litres d'acide chlorhydrique 0,012 N, sous agitation à une température comprise entre environ 4°C et 8°C, pendant 8 heures au minimum.

La solution est ensuite filtrée stérilement sur un filtre plaque et le filtrat est récupéré dans un flacon stérile.

On additionne alors au filtrat, sous agitation, 4,1 litres de chlorure de sodium (240 g/l) stérile et on poursuit l'agitation pendant environ 10 minutes. On laisse au contact pendant 8 heures minimum.

La suspension est alors transférée, dans des conditions stériles, dans des récipients de centrifugation stériles. On procède à une centrifugation à environ 8000 t.p.m. pendant 15 minutes à 15°C environ.

On récupère le précipité pour procéder à l'oxydation ménagée par l'acide périodique.

Pour cela, le précipité est repris dans de l'acide chlorhydrique 0,012 N stérile en ajustant le concentration en collagène à 0,5 %. On agite à une température comprise entre environ 4°C et 8°C pendant 8 heures minimum.

On ramène ensuite la température du milieu à environ 20°C puis on y ajoute, sous agitation, 80 ml d'acide périodique 0,4 M stérile (365 mg d'acide périodique par gramme de collagène).

On poursuit l'agitation pendant environ 2 heures à l'abri de la lumière et on ajoute alors 0,8 litre de chlorure de sodium (240 g/l) stérile, en maintenant l'agitation environ 5 minutes supplémentaires.

On laisse reposer la suspension environ 30 minutes puis on la transfère dans des récipients de centrifugation stériles. On procède alors à la centrifugation à environ 8500 t.p.m. pendant 15 minutes à 20°C environ.

On récupère le précipité et on le remet en suspension dans 4800 ml (q.s.p.) de NaCl 41 g/l, HCl 0,012 N, stériles. On maintient l'agitation environ 1 heure puis on transfère la suspension dans des récipients de centrifugation stériles et on effectue la centrifugation à environ 8500 t.p.m. pendant 15 minutes à 20°C environ.

On récupère le précipité et on procède à un second lavage de la même manière que précédemment.

On récupère le précipité issu de ce second lavage et on le remet à nouveau en suspension comme pour les lavages précédents.

On maintient alors la suspension sous agitation pendant 8 heures minimum à environ 4-8°C.

La suspension est ensuite transférée dans des récipients de centrifugation et on réalise la centrifugation à 8500 t.p.m. pendant 15 minutes à 20°C.

Le précipité récupéré est remis en suspension dans une solution d'acétone à 90 % dans l'eau.

On agite environ 15 minutes et on récupère le précipité par filtration sur toile de nylon.

On lave le précipité obtenu 3 fois avec de l'acétone à 80 % et 3 fois avec de l'acétone à 100 %.

On déshydrate le précipité acétonique résultant sous courant d'air stérile jusqu'à point constant. On obtient un rendement quantitatif proche de 100 %.

Pour conserver la poudre de collagène oxydé obtenue, on la place dans des flacons stériles que l'on congèle à -80°C.

**Exemple 2 :** Préparation d'une solution acide réactive de collagène modifié par coupure oxydative à l'aide d'acide périodique et non réticulé.

On dissout la quantité nécessaire de poudre de collagène oxydé, non réticulé, dans de l'eau ultrafiltrée stérile.

La quantité de poudre ($P_p$) nécessaire à la préparation de 100 g ($P_s$) de solution acide réactive est calculée de la façon suivante :

$$P_p = \frac{Ps \times c}{(100 - h)}$$

où Ps représente la quantité de solution acide réactive préparée, c représente la concentration finale du collagène dans la solution acide réactive et h représente l'humidité résiduelle de la poudre de collagène oxydé, non réticulé.

La quantité d'eau ultrafiltrée ($P_e$) est calculée comme suit :

$$P_e = P_s - P_p$$

Ainsi, pour la préparation de 100 g d'une solution acide réactive à 15 % à partir d'une poudre ayant une humidité résiduelle de 14,5 % :

$$P_p = 17,54 \text{ g et } P_e = 82,46 \text{ g}$$

Pour la préparation de 100 g d'une solution acide réactive à 20 % à partir de la même poudre :

$$P_p = 23,39 \text{ g et } P_e = 76,61 \text{ g}$$

Pour cela, on ajuste la température de l'eau ultrafiltrée à environ 60°C et on additionne, sous agitation, la poudre de collagène obtenue conformément à l'exemple 1 par fraction d'environ un tiers à chaque fois. Entre chaque ajout, on poursuit l'agitation jusqu'à l'obtention d'une solution fluide.

Compte tenu du chauffage contrôlé opéré, le collagène perd sa structure hélicoïdale et se transforme en gélatine.

Lorsque la totalité de la poudre de collagène oxydé a été additionnée, on maintient encore l'agitation jusqu'à l'obtention d'une solution homogène légèrement visqueuse.

Pour conserver la solution acide réactive ainsi préparée, on la place dans des flacons stériles et on la congèle à -20°C.

**Exemple 3 :** Préparation d'un kit adhésif à usage chirurgical.

Pour un kit sous la forme d'une double seringue munie d'un mélangeur, on place une solution acide réactive de collagène oxydé, non réticulé, préparée conformément à l'exemple 2, dans une seringue et on place, dans l'autre seringue, une solution tampon.

Pour 100 g de solution acide réactive à 15 %, il y a 27,5 g de tampon carbonate de sodium 0,41 M.

## II - TEST DE REACTIVITE IN VITRO.

On mesure le temps nécessaire à la réticulation de la solution acide réactive conduisant à un gel, dans les conditions physiologiques d'utilisation suivantes :

- On chauffe 1 g de solution acide réactive à 15 % à environ 42 °C pendant 2 à 3 minutes ;
- On y ajoute 275 microlitres de tampon carbonate de sodium 0,41 M ;
- On agite le mélange à la spatule pendant environ 15 secondes.
- On note l'aspect du produit résultant toute les 60 secondes.

Les résultats sont les suivants :

| Etape I | après 0,5 min | aspect visqueux |
|---|---|---|
| Etape II | après 1,5 min | aspect semi-gélifié |
| Etape III | après 2,5 min | aspect gélifié solide |

## III - TEST D'ADHESIVITE : EVALUATION EX VIVO.

L'évaluation des propriétés adhésives de compositions selon l'invention a été réalisée sur des tissus musculaires de lapin (râble). Ces tissus sont conservés à 4°C dans du sérum physiologique pendant 48 heures au maximum. Le tissu de lapin est découpé dans le sens des fibres à l'aide d'une trancheuse électrique (épaisseur des tranches : 2,5 ±0,5 mm), puis des carrés de 25 mm x 25 mm sont découpés dans les tranches obtenues.

Les essais sont réalisés sur un appareil de traction usuel, par exemple de type Adamel Lhomargy de type DY34 muni d'un capteur de force de 100 N. Cet appareil permet d'obtenir les courbes force-déplacement. Il permet également d'obtenir la force maximale d'arrachement ($F_{max}$), le module de Young, et l'énergie mise en jeu peut être calculée d'après l'aire sous la courbe.

Dans chaque type d'essai, deux éprouvettes de tissu de lapin sont fixées à l'aide d'une colle cyanoacrylique (par exemple vendue sous la marque "Loctite superglue", liquide ou gel) sur des supports inertes, verres ou cartons très rigides de dimension supérieure. Les tests sont réalisés au bout de 3 minutes après une pression de 1 ou 4 N.

La composition est appliquée sur les tissus par un système d'application double seringue à raison de 400 µl par échantillon selon l'exemple 3, à une concentration en collagène de 15 %.

On enregistre après 3 minutes de contact avec :

a) la composition adhésive, sous 4 N :

- une force adhésive maximale moyenne de 2,5 N (±0,36) ; (n=3)
- une énergie d'adhésion moyenne de 10,1 mJ (±3,7) ; (n=3)

b) la composition adhésive, sous 1 N :

- une force adhésive maximale moyenne de 3,2 N (±1,7) ; (n=3)
- une énergie d'adhésion moyenne de 5,4 mJ (±3,3) ; (n=3)

c) la colle de Fibrine, sous 4 N :

- une force adhésive maximale moyenne de 2,5 N (±1,6) ;
- une énergie d'adhésion moyenne de 3,8 mJ (±2).

Ces valeurs montrent que la composition adhésive selon l'invention donne des valeurs d'adhésion ex vivo au moins égales à celles de la colle de fibrine utilisée comme référence.

## IV - TEST D'ADHESIVITE IN VIVO.

Le pouvoir adhésif de la composition selon l'invention est déterminé in vivo dans un modèle standardisé de collage de lambeau de peau.

Sur des rats de type OFA ou Sprague Dawley préalablement anesthésiés et tondus, deux plaies équivalentes sont réalisées sur chaque flanc du rat par rapport à la ligne médiane de la colonne vertébrale. La plaie type comporte trois cotés de 2 à 3 cm de longueur. Les incisions sont réalisées sur les côtés têtes, dos et queue de chaque flanc. L'incision tête étant réalisée à environ 13 cm du bout du museau du rat.

Après réalisation de la plaie type, 0,2 à 0,4 ml de composition selon l'invention est appliqué sur la plaie par un système d'application double seringue ; puis le lambeau de peau est positionné et maintenu en place par le manipulateur pendant 1 à 2 minutes. Les berges de la plaie, correctement maintenues par le produit selon l'invention, ne se détachent alors plus et restent fermement maintenues. En l'absence de produit ou en présence d'un autre type de collagène tel que du collagène chauffé non oxydé, les berges des plaies ne restent pas correctement en place et la plaie s'ouvre à nouveau.

La plaie controlatérale est collée de manière identique par de la colle de fibrine (TISSUCOL ou BIOCOL) servant de produit de référence.

Ce test caractérise d'une part, l'adhésivité primaire de l'adhésif selon l'invention, à savoir l'efficacité de collage au moment de la mise en place qui doit permettre un maintien des berges en place :

- En situation per opératoire, elle est observée après 1 à 2 minutes de maintien par le manipulateur ;
- En situation postopératoire, elle est observée dans les deux premières heures lorsque l'animal est anesthésié, et dans les deux premiers jours lorsque l'animal est vigile et bouge.

Par ailleurs, le maintien primaire doit être relayé par le processus de cicatrisation normal des tissus conduisant à l'adhésion définitive des berges de la plaie que l'on qualifie d'adhésivité secondaire.

Ce test caractérise d'autre part, le bon maintien des berges accolées qui est observé après le réveil de l'animal puis à 24, 48 et 72 heures.

- Si le produit ne permet pas d'obtenir une adhésion physiologiquement correcte, le lambeau de peau se détache et conduit à une ouverture de la plaie.
- Si le produit conduit à une adhésion primaire puis secondaire correctes, le lambeau de peau reste correctement accroché et la cicatrisation définitive se poursuit.

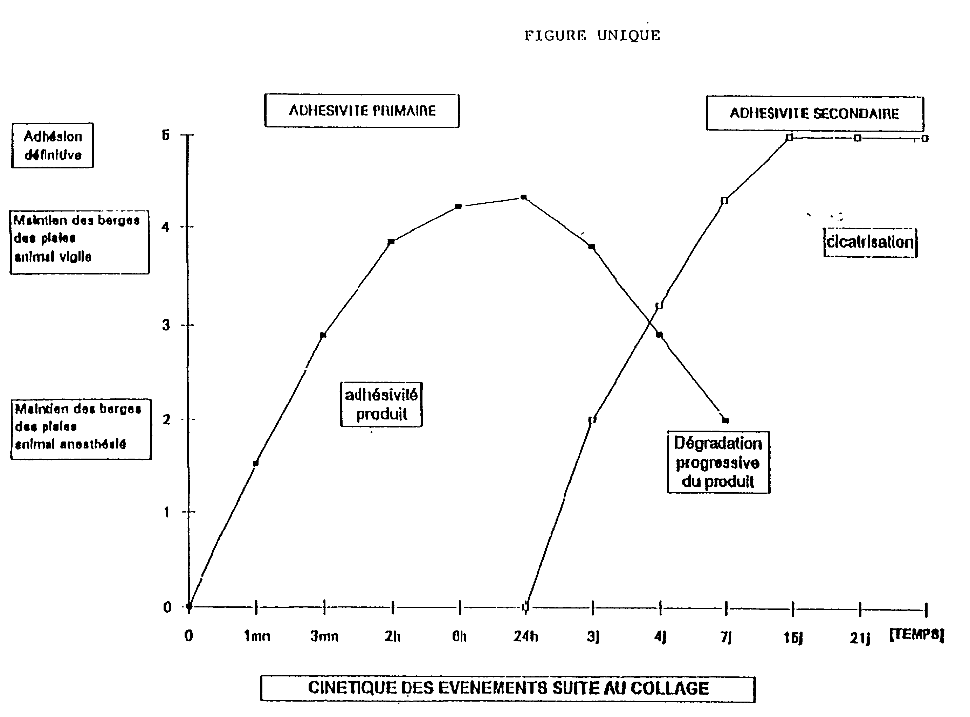

La figure 1 illustre la cinétique des évènements se produisant après application de l'adhésif selon l'invention.

Un test comparatif a été effectué avec la colle de fibrine :

Lors du test, le nombre de berges de plaies qui demeurent correctement collées est compté tous les jours durant 3 jours.

Le résultat est exprimé en nombre de plaies correctement collées en fonction du nombre total de plaies.

Les deux tableaux suivants montrent les résultats de deux essais indépendants, l'un conduit avec comme référence TISSUCOL et l'autre conduit avec comme référence BIOCOL.

Les résultats montrent que l'adhésion observée avec la composition adhésive sur 3 jours est au moins égale aux colles de référence BIOCOL et TISSUCOL.

En l'absence de produit adhésif, aucune plaie ne reste correctement collée.

|  | J1 | J2 | J3 |
|---|---|---|---|
| COMPOSITION ADHESIVE EXEMPLE 3 | 49/51 | 44/51 | 34/51 |
| TISSUCOL | 51/51 | 46/51 | 39/51 |

Essai sur 17 rats.

|  | J1 | J2 | J3* |
|---|---|---|---|
| COMPOSITION ADHESIVE EXEMPLE 3 | 24/24 | 23/24 | 15/21 |
| BIOCOL | 17/24 | 16/24 | 15/21 |

Essai sur 8 rats (* 1 rat est décédé à J3 sans relation avec le produit, supprimant l'observation de 3 berges).

Ce test met ainsi en évidence que l'adhésif selon l'invention permet d'obtenir une adhésion aussi satisfaisante que la colle de fibrine.

**Revendications**

1.  Composition adhésive biocompatible, biorésorbable et non toxique, à usage chirurgical notamment pour la liaison de tissus biologiques entre eux ou avec un biomatériau implanté, caractérisée en ce qu'elle comprend une solution acide réactive de collagène ou de gélatine modifié(e) par coupure oxydative, non réticulé(e) et potentiellement réticulable.

2.  Composition adhésive selon la revendication 1, caractérisée en ce que la solution acide réactive est constituée de collagène ou de gélatine oxydé(e) à l'aide d'une solution d'acide périodique ou un de ses sels, à une concentration comprise entre 1 et $10^{-5}$ M.

3.  Composition adhésive selon l'une quelconque des revendications 1 et 2, caractérisé en ce que la concentration en collagène ou en gélatine est comprise entre 5 et 30 % en poids.

4.  Composition adhésive selon l'une quelconque des revendications 1 à 3, caractérisée en ce que la solution acide réactive est conservée à une température inférieure à 0°C.

5.  Composition adhésive selon la revendication 4, caractérisée en ce que ladite solution acide réactive est conservée à une température comprise entre -10°C et -80°C .

6.  Composition adhésive selon l'une quelconque des revendications précédentes, caractérisée en ce que le collagène ou la gélatine est d'origine humaine ou animale.

7.  Composition adhésive selon l'une quelconque des revendications précédentes, caractérisée en ce que la solution acide réactive est une solution concentrée à pH acide dans de l'eau stérile, de collagène ou de gélatine oxydé(e) par l'acide périodique ou l'un de ses sels et déshydraté(e) sous forme de poudre.

8.  Poudre de collagène ou de gélatine modifié(e) par coupure oxydative, non réticulé(e) et potentiellement réticulable, soluble à pH acide.

9.  Poudre selon la revendication 8, caractérisée en ce quelle comprend du collagène ou de la gélatine oxydé(e) à l'aide d'une solution d'acide périodique ou un de ses sels, à une concentration comprise entre 1 et $10^{-5}$ M.

10. Poudre selon l'une quelconque des revendications 8 et 9, caractérisée en ce qu'elle est conservée à une température inférieure à 0°C.

11. Poudre selon la revendication 10, caractérisée en ce qu'elle est conservée à une température comprise entre -10°C et -80 °C.

12. Poudre selon l'une quelconque des revendications 8 à 11, caractérisée en ce que le collagène ou la gélatine est d'origine humaine ou animale.

13. Procédé de préparation d'une poudre selon l'une quelconque des revendications 8 à 12, caractérisé en ce qu'il consiste à :

- préparer une solution acide de collagène ;
- soumettre ladite solution aqueuse acide et à tempe rature ambiante, à une oxydation ménagée par une solution d'acide périodique ou un de ses sels, à une concentration comprise entre 1 et $10^{-5}$ M ;
- précipiter le collagène oxydé et non réticulé, à pH acide ; et
- isoler et concentrer puis déshydrater ledit collagène modifié par coupure oxydative et non réticulé, pour l'obtenir sous forme de poudre acide réactive.

14. Procédé selon la revendication 13, caractérisé en ce que l'on précipite à pH acide, le collagène oxydé et non réticulé par addition de sel, notamment de chlorure de sodium.

15. Procédé selon l'une quelconque des revendications 13 et 14, caractérisé en ce que l'on utilise du collagène d'origine humaine ou animale.

16. Solution acide réactive concentrée de collagène ou de gélatine modifié(e) par coupure oxydative, non réticulé(e) et potentiellement réticulable, pour former une composition adhésive.

17. Solution acide réactive selon la revendication 16, caractérisée en ce qu'elle est préparée à partir de la poudre selon l'une quelconque des revendications 8 à 12 par dissolution dans l'eau, à pH acide.

18. Solution selon l'une quelconque des revendications 16 et 17, caractérisée en ce que la concentration en collagène ou gélatine est comprise entre 5 et 30 % en poids.

19. Solution selon l'une quelconque des revendications 16 à 18, caractérisée en ce qu'elle est conservée à une température inférieure à 0°C.

20. Solution selon l'une quelconque des revendications 16 à 19, caractérisée en ce qu'elle est conservée à une température comprise entre -10°C et -80 °C.

21. Procédé de préparation d'une solution acide réactive selon la revendication 17, caractérisé en ce qu'il consiste à :

- préparer une poudre stérile de collagène ou de gélatine modifié(e) par coupure oxydative, non réticulé(e) et potentiellement réticulable selon l'une quelconque des revendications 13 à 15,
- dissoudre dans de l'eau stérile la quantité nécessaire de cette poudre, en chauffant à une température comprise entre 40°C et 80°C.

22. Procédé selon la revendication 21, caractérise en ce que l'on dissout dans de l'eau stérile la poudre de collagène ou de gélatine modifié(e) par coupure oxydative et non réticulé(e), par quantité d'environ 5 % (poids/poids).

23. Kit adhésif biocompatible, biorésorbable et non toxique, destiné à un usage chirurgical notamment pour la liaison de tissus biologiques entre eux ou avec un biomatériau implanté, caractérisé en ce qu'il comprend :

- une solution acide réactive de collagène ou de gélatine modifié(e) par coupure oxydative, non réticulé(e) et potentiellement réticulable,
- une solution de neutralisation, et
- des moyens de mélange pour mélanger extemporanément lesdites solutions.

24. Kit selon la revendication 23, caractérisé en ce que la solution acide réactive est une solution selon l'une quelconque des revendications 16 à 20.

**25.** Kit selon l'une quelconque des revendications 23 et 24, caractérisé en ce que la solution acide réactive est conservée à une température inférieure à 0°C.

**26.** Kit selon la revendication 25, caractérisé en ce que la solution acide réactive est conservée à une température comprise entre -10°C et - 80°C.

**27.** Kit selon l'une quelconque des revendications 23 à 26, caractérisé en ce que la solution acide réactive est obtenue par le procédé selon l'une quelconque des revendications 21 et 22.

**28.** Kit selon l'une quelconque des revendications 23 à 27, caractérisé en ce que la solution de neutralisation est un tampon permettant d'obtenir, après mélange avec la solution acide réactive, un pH compris entre 6 et 10.

**29.** Kit selon la revendications 28, caractérisé en ce que ledit tampon est constitué de carbonate ou de phosphate de sodium.

**30.** Kit selon l'une quelconque des revendications 23 à 29, caractérisé en ce qu'il se présente sous la forme d'une double seringue équipée de moyens de mélange, dont l'une des seringues contient la solution acide réactive de collagène ou de gélatine modifié(e) par coupure oxydative, non réticulé(e) et potentiellement réticulable, et l'autre contient la solution de neutralisation.

**31.** Procédé d'application in vitro sur des tissus biologiques et/ou un biomatériau, d'une composition adhésive selon l'une quelconque des revendications 1 à 7, caractérisé en ce qu'il consiste à :

- amener la solution acide réactive de collagène ou de gélatine modifié(e) par coupure oxydative et non réticulé (e), à un état de fluidité adapté à une répartition convenable sur lesdits tissus et/ou biomatériau ;
- mélanger extemporanément la solution acide réactive de collagène ou de gélatine et une solution de neutralisation de manière à obtenir un pH compris entre 6 et 10 ; appliquer immédiatement avant réticulation, le mélange réactif neutralisé et présentant un état de fluidité adapté, sur les tissus biologiques et/ou biomatériau à lier ;
- laisser polymériser par réticulation à la température du corps humain.

**32.** Procédé d'application selon la revendication 31, caractérisé en ce que, pour atteindre ledit état de fluidité adapté, on chauffe la solution acide réactive à une température comprise entre 30°C et 60°C et on applique sur lesdits tissus et/ou biomatériau, ladite solution acide réactive chauffée après neutralisation.

**33.** Procédé d'application selon la revendication 3 caractérisé en ce que l'on chauffe la solution acide réactive à environ 37°C.

**34.** Procédé d'application selon la revendication 31 caractérisé en ce que la solution de neutralisation est un tampon carbonate ou phosphate de sodium.

**35.** Utilisation d'une composition adhésive selon l'une quelconque des revendications 1 à 7 pour la préparation d'un matériau adhésif biocompatible, biorésorbable et non toxique, constitué de collagène ou de gélatine réticulé(e), dans la masse et de manière homogène.

**Patentansprüche**

**1.** Biokompatibler, bioresorbierbarer und ungiftiger Klebstoff zur Verwendung in der Chirurgie, insbesondere für die Verbindung von biologischen Geweben untereinander oder mit einem implantierten Biomaterial, dadurch gekennzeichnet, daß er eine saure reaktionsfähige Lösung von durch oxydative Spaltung modifiziertem(er), nicht vernetztem(er) und potentiell vernetzbarem(er) Kollagen oder Gelatine enthält.

**2.** Klebstoff gemäß Anspruch 1, dadurch gekennzeichnet, daß die saure reaktionsfähige Lösung aus mit Hilfe einer Lösung aus Periodsäure oder einem ihrer Salze in einer Konzentration zwischen 1 und $10^{-5}$ M oxydiertem(er) Kollagen oder Gelatine besteht.

3. Klebstoff gemäß einem der Ansprüche 1 und 2, dadurch gekennzeichnet, daß die Konzentration an Kollagen oder Gelatine zwischen 5 und 30 Gew.% beträgt.

4. Klebstoff gemäß einem der Ansprüche 1 bis 3, dadurch gekennzeichnet, die saure reaktionsfähige Lösung bei einer Temperatur unter 0°C aufbewahrt wird.

5. Klebstoff gemäß Anspruch 4, dadurch gekennzeichnet, daß die besagte saure reaktionsfähige Lösung bei einer Temperatur zwischen -10°C und -80°C aufbewahrt wird.

6. Klebstoff gemäß einem der vorhergehenden Ansprüche, dadurch gekennzeichnet, daß das Kollagen oder die Gelatine menschlicher oder tierischer Herkunft ist.

7. Klebstoff gemäß einem der vorhergehenden Ansprüche, dadurch gekennzeichnet, daß die saure reaktionsfähige Lösung eine konzentrierte, in sterilem Wasser mit saurem pH vorliegende Lösung von durch Periodsäure oder eines ihrer Salze oxydiertem(er) und entwässertem(er) und somit in Pulverform vorliegendem(er) Kollagen oder Gelatine ist.

8. Pulver von durch oxydative Spaltung modifiziertem(er), nicht vernetztem(er) und potentiell vernetzbarem(er), bei saurem pH löslichem(er) Kollagen oder Gelatine.

9. Pulver gemäß Anspruch 8, dadurch gekennzeichnet, daß es Kollagen oder Gelatine enthält, das(die) mit Hilfe einer Lösung von Periodsäure oder einem ihrer Salze in einer Konzentration zwischen 1 und $10^{-5}$M oxydiert wurde.

10. Pulver gemäß einem der Ansprüche 8 und 9, dadurch gekennzeichnet, daß es bei einer Temperatur unter 0°C aufbewahrt wird.

11. Pulver gemäß Anspruch 10, dadurch gekennzeichnet, daß es bei einer Temperatur zwischen -10°C und -80°C aufbewahrt wird.

12. Pulver gemäß einem der Ansprüche 8 bis 11, dadurch gekennzeichnet, daß das Kollagen oder die Gelatine menschlicher oder tierischer Herkunft ist.

13. Verfahren zur Herstellung eines Pulvers gemäß einem der Ansprüche 8 bis 12, dadurch gekennzeichnet, daß es besteht in

- der Herstellung einer sauren Kollagenlösung;

- einer schonend durchgeführten Oxidation der besagten wäßrigen, sauren und bei Raumtemperatur vorliegenden Lösung durch eine Lösung von Periodsäure oder einem ihrer Salze in einer Konzentration zwischen 1 und $10^{-5}$ M;

- der Fällung des oxydierten und nicht vernetzten Kollagens bei saurem pH; und

- der Isolierung, Konzentration und anschließenden Dehydratisierung des besagten, durch oxydative Spaltung modifizierten und nicht vernetzten Kollagens, um es in Form eines sauren, reaktionsfähigen Pulvers zu erhalten.

14. Verfahren gemäß Anspruch 13, dadurch gekennzeichnet, daß das oxydierte und nicht vernetzte Kollagen durch Zugabe von Salz, vor allem von Natriumchlorid, bei saurem pH gefällt wird.

15. Verfahren gemäß einem der Ansprüche 13 und 14, dadurch gekennzeichnet, daß man Kollagen menschlicher oder tierischer Herkunft verwendet.

16. Saure reaktionsfähige konzentrierte Lösung von durch oxydative Spaltung modifiziertem(er), nicht vernetztem(er) und potentiell vernetzbarem(er) Kollagen oder Gelatine für die Bildung eines Klebstoffs.

17. Saure reaktionsfähige Lösung gemäß Anspruch 16, dadurch gekennzeichnet, daß sie aus dem Pulver gemäß einem der Ansprüche 8 bis 12 durch Auflösen in Wasser bei saurem pH gebildet wird.

**18.** Lösung gemäß einem der Ansprüche 16 und 17, dadurch gekennzeichnet, daß die Konzentration an Kollagen oder Gelatine zwischen 5 und 30 Gew.% liegt.

**19.** Lösung gemäß einem der Ansprüche 16 bis 18, dadurch gekennzeichnet, daß sie bei einer Temperatur unter 0°C aufbewahrt wird.

**20.** Lösung nach einem der Ansprüche 16 bis 19, dadurch gekennzeichnet, daß sie bei einer Temperatur zwischen -10°C und -80°C aufbewahrt wird.

**21.** Verfahren zur Herstellung einer sauren reaktionsfähigen Lösung gemäß Anspruch 17, dadurch gekennzeichnet, daß es besteht in

- der Herstellung eines sterilen Pulvers aus durch oxydative Spaltung modifiziertem(er), nicht vernetztem(er) und potentiell vernetzbarem(er) Kollagen oder Gelatine gemäß einem der Ansprüche 13 bis 15;

- der Auflösung der erforderlichen Menge dieses Pulvers in sterilem Wasser durch Erwärmen auf eine Temperatur zwischen 40°C und 80°C.

**22.** Verfahren gemäß Anspruch 21, dadurch gekennzeichnet, daß man das Pulver aus durch oxydative Spaltung modifiziertem(er) und nicht vernetztem(er) Kollagen oder Gelatine in einer Menge von etwa 5 % (Gew./Gew.) in sterilem Wasser auflöst.

**23.** Biokompatibles, bioresorbierbares und ungiftiges Klebstoff-Set zur Verwendung in der Chirurgie, insbesondere für die Verbindung biologischer Gewebe untereinander oder mit einem implantierten Biomaterial, dadurch gekennzeichnet, daß es umfaßt

- eine saure, reaktionsfähige Lösung von durch oxydative Spaltung modifiziertem(er), nicht vernetztem(er) und potentiell vernetzbarem(er) Kollagen oder Gelatine,

- eine Neutralisationslösung und

- Mischungsmittel zum Mischen der besagten Lösungen während der Operation.

**24.** Set gemäß Anspruch 23, dadurch gekennzeichnet, daß die saure reaktionsfähige Lösung eine Lösung gemäß einem der Ansprüche 16 bis 20 ist.

**25.** Set gemäß einem der Ansprüche 23 und 24, dadurch gekennzeichnet, daß die saure reaktionsfähige Lösung bei einer Temperatur unter 0°C aufbewahrt wird.

**26.** Set gemäß Anspruch 25, dadurch gekennzeichnet, daß die saure reaktionsfähige Lösung bei einer Temperatur zwischen -10°C und -80°C aufbewahrt wird.

**27.** Set gemäß einem der Ansprüche 23 bis 26, dadurch gekennzeichnet, daß die saure reaktionsfähige Lösung durch das Verfahren gemäß einem der Ansprüche 21 und 22 erhalten wird.

**28.** Set gemäß einem der Ansprüche 23 bis 27, dadurch gekennzeichnet, daß die Neutralisationslösung ein Puffer ist, mit dem man nach der Mischung mit der sauren reaktionsfähigen Lösung einen pH-Wert zwischen 6 und 10 erhalten kann.

**29.** Set gemäß Anspruch 28, dadurch gekennzeichnet, daß der Puffer aus Natriumcarbonat oder -phosphat besteht.

**30.** Set gemäß einem der Ansprüche 23 bis 29, dadurch gekennzeichnet, daß es in Form einer mit Mischungsmitteln ausgestatteten Zwillingsspritze vorliegt, von denen eine die saure reaktionsfähige Lösung von durch oxydative Spaltung modifiziertem(er), nicht vernetztem(er) und potentiell vernetzbarem(er) Kollagen oder Gelatine, und die andere die Neutralisationslösung enthält.

**31.** Verfahren zum Auftragen eines Klebstoffs gemäß einem der Ansprüche 1 bis 7 in vitro auf biologische Gewebe und/oder ein Biomaterial, dadurch gekennzeichnet, daß es besteht in:

- der Überführung der sauren reaktionsfähigen Lösung von durch oxydative Spaltung modifiziertem(er) und nicht vernetztem(er) Kollagen oder Gelatine in einen Zustand der Liquidität, der einer zweckentsprechenden Verteilung auf den besagten Geweben und/oder dem Biomaterial angepaßt ist;

- der während der Operation vorgenommenen Mischung der sauren reaktionsfähigen Kollagen- oder Gelatinelösung mit einer Neutralisationslösung, um einen pH-Wert zwischen 6 und 10 zu erhalten; der unmittelbar vor der Vernetzung erfolgten Auftragung der reaktionsfähigen, neutralisierten und einen geeigneten Liquiditätszustand aufweisenden Mischung auf die zu verbindenden biologischen Gewebe und/oder das Biomaterial;

- der Polymerisation durch Vernetzung bei der Körpertemperatur des Menschen.

32. Auftragungsverfahren gemäß Anspruch 31, dadurch gekennzeichnet, daß man zur Erreichung des besagten geeigneten Liquiditätszustandes die saure reaktionsfähige Lösung auf eine Temperatur zwischen 30°C und 60°C erwärmt und die besagte saure reaktionsfähige erwärmte Lösung nach der Neutralisation auf die besagten Gewebe und/oder das Biomaterial aufträgt.

33. Auftragungsverfahren gemäß Anspruch 31, dadurch gekennzeichnet, daß man die saure reaktionsfähige Lösung auf etwa 37°C erwärmt.

34. Auftragungsverfahren gemäß Anspruch 31, dadurch gekennzeichnet, daß die Neutralisationslösung ein Natriumcarbonat- oder Natriumphosphat-Puffer ist.

35. Verwendung eines Klebstoffs nach einem der Ansprüche 1 bis 7 zur Herstellung eines biokompatiblen, bioresorbierbaren und ungiftigen klebenden Materials, bestehend aus vernetztem(er) Kollagen oder Gelatine, in der Masse und in homogener Form.


**Claims**

1. Adhesive composition which is biocompatible, bioresorbable and non-toxic for surgical use, in particular for joining biological tissues to each other or to an implanted biomaterial, characterised in that said composition comprises a reactive acidic solution of collagen or of gelatine which is modified by oxidative degradation, is non-cross-linked and potentially cross-linkable.

2. Adhesive composition according to claim 1, characterised in that the reactive acidic solution is formed from collagen or gelatine which is oxidised with the help of a periodic acidic solution or of one of its salts, in a concentration between 1 and $10^{-5}$ M.

3. Adhesive composition according to any of the claims 1 and 2, characterised in that the concentration of collagen or gelatine is between 5 and 30% by weight.

4. Adhesive composition according to any of the claims 1 to 3, characterised in that the reactive acidic solution is preserved at a temperature below 0°C.

5. Adhesive composition according to claim 4 characterised in that said reactive acidic solution is preserved at a temperature between -10°C and -80°C.

6. Adhesive composition according to any of the preceding claims, characterised in that the collagen or the gelatine is of human or animal origin.

7. Adhesive composition according to any of the preceding claims, characterised in that the reactive acidic solution is a concentrated solution, with an acidic pH in sterile water, of collagen or gelatine which is oxidised by periodic acid or one of its salts and dehydrated in the form of a powder.

8. Powder of collagen or gelatine which is modified by oxidative degradation, non-cross-linked and potentially cross-linkable, and soluble with an acidic pH.

9. Powder according to claim 8, characterised in that it contains collagen or gelatine which is oxidised with the help of a solution of periodic acid or one of its salts at a concentration between 1 and $10^{-5}$ M.

10. Powder according to any of the claims 8 and 9, characterised in that it is preserved at a temperature below 0°C.

11. Powder according to claim 10, characterised in that it is preserved at a temperature between -10°C and -80°C.

12. Powder according to any of the claims 8 to 11, characterised in that the collagen or gelatine is of human or animal origin.

13. Method for preparing a powder according to any of the claims 8 to 12, characterised in that it comprises :

- preparing an acidic solution of collagen,

- subjecting said aqueous acidic solution at ambient temperature to oxidation provided by a solution of periodic acid or by one of its salts at a concentration between 1 and $10^{-5}$ M;

- precipitating the oxidised, non-cross-linked collagen at an acidic pH; and

- isolating and concentrating then dehydrating said collagen which is modified by oxidative degradation and is non-cross-linked in order to obtain it in the form of a reactive acidic powder.

14. Method according to claim 13, characterised in that the oxidised, non-cross-linked collagen is precipitated at an acidic pH by adding a salt, particularly sodium chloride.

15. Method according to any of the claims 13 and 14, characterised in that the collagen used is of human or animal origin.

16. Concentrated reactive acidic solution of collagen or gelatine which is modified by oxidative degradation, non-cross-linked and potentially cross-linkable in order to form an adhesive composition.

17. Reactive acidic solution according to claim 16, characterised in that it is prepared from the powder according to any of the claims 8 to 12 by dissolving in water at an acidic pH.

18. Solution according to any of the claims 16 and 17, characterised in that the concentration of collagen or gelatine is between 5 and 30% by weight.

19. Solution according to any of the claims 16 to 18, characterised in that it is preserved at a temperature below 0°C.

20. Solution according to any of the claims 16 to 19, characterised in that it is preserved at a temperature between -10°C and -80°C.

21. Method for preparing a reactive acidic solution according to claim 17, characterised in that it comprises :

- preparing a sterile powder of collagen or gelatine which is modified by oxidative degradation, non-cross-linked and potentially cross-linkable according to any of the claims 13 to 15,

- dissolving the necessary quantity of this powder in sterile water by heating to a temperature between 40°C and 80°C.

22. Method according to claim 21, characterised in that the powder of collagen or gelatine which is modified by oxidative degradation and non-cross-linked is dissolved in a quantity of approx. 5% (weight/weight) in sterile water.

23. Adhesive kit which is biocompatible, bioresorbable and non-toxic and intended for surgical use, in particular for joining biological tissues to each other or to an implanted biomaterial, characterised in that it comprises :

- a reactive acidic solution of collagen or gelatine which is modified by oxidative degradation, non-cross-linked and potentially cross-linkable,

- a neutralising solution, and

- mixing means for mixing said solutions extemporaneously.

24. Kit according to claim 23, characterised in that the reactive acidic solution is a solution according to any of the claims 16 to 20.

25. Kit according to any of the claims 23 and 24, characterised in that the reactive acidic solution is preserved at a temperature below 0°C.

26. Kit according to claim 25, characterised in that the reactive acidic solution is preserved at a temperature between -10°C and -80°C.

27. Kit according to any of the claims 23 to 26, characterised in that the reactive acidic solution is obtained by the method according to any of the claims 21 and 22.

28. Kit according to any of the claims 23 to 27, characterised in that the neutralising solution is a buffer which allows a pH between 6 and 10 to be obtained after mixing with the reactive acidic solution.

29. Kit according to claim 28, characterised in that said buffer is made of sodium carbonate or sodium phosphate.

30. Kit according to any of the claims 23 to 29, characterised in that it appears in the form of a double syringe provided with mixing means, one of which syringes contains the reactive acidic solution of collagen or gelatine which is modified by oxidative degradation, non-cross-linked and potentially cross-linkable, and the other contains the neutralising solution.

31. Method for application in vitro on biological tissues and/or a biomaterial of an adhesive composition according to any of the claims 1 to 7, characterised in that it comprises :

- bringing the reactive acidic solution of collagen or gelatine, which is modified by oxidative degradation and non-cross-linked, to a state of fluidity suitable for appropriate distribution on said tissues and/or biomaterial;

- mixing the reactive acidic solution of collagen or gelatine and a neutralising solution extemporaneously so as to obtain a pH between 6 and 10; applying the neutralised reactive mixture which is displaying an adapted state of fluidity, immediately before cross-linkage, on the biological tissues and/or the biomaterial to be joined;

- leaving to polymerise by cross-linkage at the temperature of the human body.

32. Method for application according to claim 31, characterised in that in order to obtain said suitable state of fluidity, the reactive acidic solution is heated to a temperature between 30°C and 60°C and said heated reactive acidic solution is applied after neutralisation on said tissues and/or biomaterial.

33. Method for application according to claim 3, characterised in that the reactive acidic solution is heated to approx. 37°C.

34. Method for application according to claim 31, characterised in that the neutralising solution is a buffer of sodium carbonate or sodium phosphate.

35. Use of an adhesive composition according to any of the claims 1 to 7 for preparing an adhesive material which is biocompatible, bioresorbable and non-toxic, made of collagen or gelatine, which is cross-linked in bulk and in a homogeneous manner.

FIGURE UNIQUE

CINETIQUE DES EVENEMENTS SUITE AU COLLAGE